(19) Europäisches Patentamt
European Patent Office
Office européen des brevets

(11) **EP 3 694 597 B1**

(12) **EUROPÄISCHE PATENTSCHRIFT**

(45) Veröffentlichungstag und Bekanntmachung des Hinweises auf die Patenterteilung:
**11.03.2026   Patentblatt 2026/11**

(21) Anmeldenummer: **18786301.4**

(22) Anmeldetag: **12.10.2018**

(51) Internationale Patentklassifikation (IPC):
**A61M 39/28** *(2006.01)*

(52) Gemeinsame Patentklassifikation (CPC):
**A61M 39/284; A61M 39/28;** A61M 2205/0272;
A61M 2205/8287

(86) Internationale Anmeldenummer:
**PCT/EP2018/077813**

(87) Internationale Veröffentlichungsnummer:
**WO 2019/073016 (18.04.2019 Gazette 2019/16)**

(54) **KLEMME**

CLAMP

PINCE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.10.2017   DE 102017218216**
**04.09.2018   DE 102018214989**

(43) Veröffentlichungstag der Anmeldung:
**19.08.2020   Patentblatt 2020/34**

(73) Patentinhaber: **Fresenius Medical Care Deutschland GmbH**
**61352 Bad Homburg (DE)**

(72) Erfinder:
• **BEISSER, Nicolas**
**63454 Hanau (DE)**
• **PETERS, Arne**
**61352 Bad Homburg (DE)**
• **HEIDE, Alexander**
**65817 Eppstein (DE)**
• **FISCHER, Gerome**
**99947 Weberstedt (DE)**

(74) Vertreter: **RCD-Patent**
**Giesen, Schmelcher & Griebel**
**Patentanwälte PartG mbB**
**Kaiserstraße 100**
**52134 Herzogenrath (DE)**

(56) Entgegenhaltungen:
EP-A1- 1 132 108      EP-B1- 0 616 155
WO-A1-2011/121923      WO-A2-2005/117240
JP-A- S5 639 370      US-A1- 2005 150 558
US-A1- 2014 318 639

## Beschreibung

### Hintergrund

In vielen Bereichen der Medizintechnik werden Klemmen eingesetzt, mit denen der Fluss durch einen fluidführenden Kanal, wie z.B. einer Schlauchleitung oder einem in eine Kassette eingelassenen Kanal, der durch eine flexible Wandung begrenzt ist, geschaltet werden kann.

Beispielhaft sind aus der US-Patentanmeldung US 2014/318639 A1 und der EP 1 132 108 A1 Geräte zur Überwachung und Regulierung eines Fluidflusses bekannt. Das Gerät weist auch ein Sicherheitsventil mit einer mechanisch anspruchsvollen Konstruktion auf.

Solche schaltbaren Klemmen weisen ein Klemmelement, ein Federelement und ein stromgesteuertes Halteelement auf. Das Federelement stellt eine Schließkraft bereit, um die Klemme im stromlosen Zustand des Halteelements zu sperren. In diesem Zustand sperrt das Klemmelement den fluidführenden Kanal in einer Geschlossen-Stellung ab und eine Fluidweiterleitung wird unterbunden.

Fluid kann in diesem Fall sowohl eine Flüssigkeit als auch ein Gas sein.

Insbesondere ist die Sperrstellung in einem stromlosen Zustand von Vorteil, da z.B. bei einem Stromausfall eine weitere (unkontrollierte) Zufuhr von Stoffen zu einem menschlichen oder tierischen Körper unterbunden sein sollte.

Das Halteelement, z.B. ein Elektromagnet, wird in einer Offenstellung der Klemmen mit Strom beaufschlagt. Das Haltelement stellt so eine Haltekraft bereit, die der Federkraft entgegenwirkt und die das Klemmelement in der Offenstellung hält.

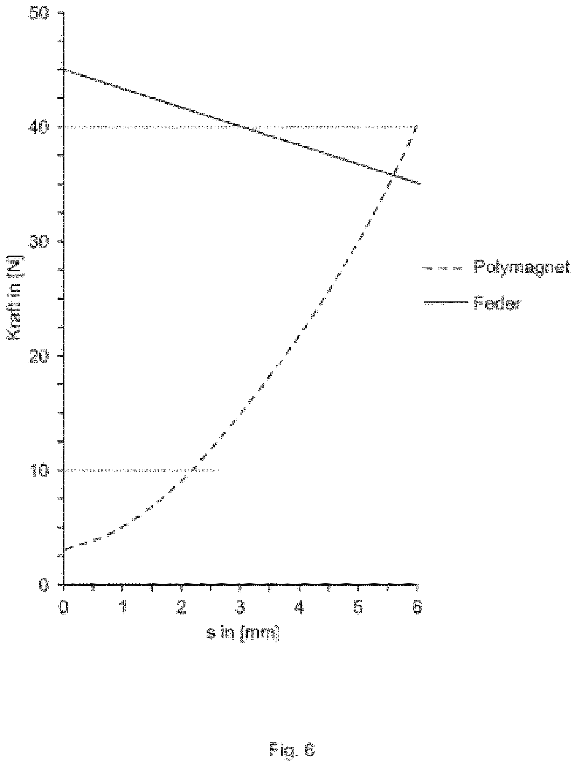

Nachteilig an dieser Anordnung ist, dass aufgrund des linearen Kraft- Weg-Verhältnisses, die Haltekraft grundsätzlich höher sein muss, als die durch das Federelement bereitgestellte Schließkraft, siehe Figur 6.

Weiterhin erfordert eine höhere Schließkraft eine entsprechend höhere Haltekraft.

Eine höhere Schließkraft erfordert ein stärker dimensioniertes und damit schweres Federelement und damit auch einen schweren Elektromagnet, höhere Halteströme, höheren Energieverbrauch.

Dies erhöht zum einen die Herstellungskosten zum anderen auch die Betriebskosten.

### Aufgabe

Es ist daher eine Aufgabe der Erfindung, eine Klemme bereitzustellen, die kostengünstiger herstellbar ist und geringere Betriebskosten aufweist. Es ist eine weitere Aufgabe der Erfindung, Klemmen bereitzustellen, deren Betrieb einen gegenüber bekannten Klemmen deutlich weniger elektrischer Energie bedarf.

### Kurzdarstellung der Erfindung

Die Aufgabe wird gelöst durch eine Klemme, welche so geartet ist, dass die Klemme einen fluidführenden Kanal aufnehmen kann, dass die Klemme in einem Ruhezustand den fluidführenden Kanal zusammendrücken kann und dass die Klemme in einem Arbeitszustand den fluidführenden Kanal so aufnehmen kann, dass der fluidführende Kanal nicht zusammengedrückt wird, wobei die Klemme einen ersten Permanentmagneten und einen zweiten Permanentmagneten aufweist, wobei die Permanentmagneten so zueinander beabstandet angeordnet sind, dass die Permanentmagneten relativ zueinander zumindest um einen Winkel drehbar sind, wobei die relative Drehung der Permanentmagneten zueinander zu einer Verringerung des Abstandes führt, wobei in einem ersten Winkel der Ruhezustand und einem zweiten Winkel der Arbeitszustand zur Verfügung gestellt wird.

Insbesondere können die Permanentmagneten relativ zueinander beweglich angeordnet sein, wobei die Permanentmagneten relativ zueinander zumindest um einen Winkel drehbar sind und wobei die Permanentmagneten linear relativ zueinander bewegbar sind, sodass der Abstand zwischen den Permanentmagneten variieren kann, wobei eine Drehung der Permanentmagneten relativ zueinander zu einer Veränderung der relativen Anordnung der Pole der Permanentmagnete zueinander führt, welche wiederum eine Veränderung der magnetischen Anziehungskräfte zwischen den Magneten bewirkt, infolgedessen eine Veränderung des Abstandes der Permanentmagnete erfolgt, wobei die Klemme in einer ersten relativen Drehstellung der Permanentmagnete zueinander einen Ruhezustand und einer zweiten relativen Drehstellung der Permanentmagnete zueinander einen Arbeitszustand einnimmt.

Ein fluidführender Kanal kann z.B. eine Schlauchleitung oder ein in eine Kassette eingelassenen Kanal, der durch eine flexible Wandung begrenzt ist, sein.

Mittels der Erfindung ist es möglich, eine Klemme bereitzustellen, die einen einfachen mechanischen Aufbau bei gleichzeitig reduzierten Halteströmen ermöglicht.

Bei einer Ausführungsform einer hier vorgeschlagenen Klemme (1), blockiert die Klemme (1) in einem Ruhezustand den Durchfluss durch zusammendrücken eines fluidführenden Kanals (L) und ermöglicht in einem Arbeitszustand durch Rücknahme des Zusammendrückens den Durchfluss. Dabei weist die Klemme (1) einen ersten Magneten (M1) und einen zweiten Magneten (M2) auf, wobei die Magneten (M1, M2) so zueinander beabstandet angeordnet sind, dass die Magneten (M1, M2) relativ zueinander zumindest um einen Winkel drehbar sind, wobei die relative Drehung der Magneten (M1, M2) zueinander zu einer Verringerung des Abstandes (d) führt, wobei in einem ersten Winkel der Ruhezustand und einem zweiten Winkel der Arbeitszustand zur Verfügung gestellt wird.

In einer Ausführungsform der Erfindung ist das Fluid eine Flüssigkeit, d.h. die Erfindung erlaubt z.B. die Zufuhr von Dialysat oder anderen Stoffen kontrolliert zu steuern.

In einer weiteren Ausführungsform der Erfindung ist ein (eingelegte) fluidführende Kanal zwischen den Permanentmagneten angeordnet.

Gemäß einer weiteren Ausführungsform der Erfindung ist der erste Permanentmagnet und/oder der zweite Permanentmagnet ein sogenannter polymagnetisierter Magnet, wobei ein solcher Permanentmagnet eine erste Anzahl von Sektoren einer ersten Magnetisierung und eine zweite Anzahl von Sektoren einer zweiten, gegensätzlichen Magnetisierung aufweist.

Mittels polymagnetisierter Magneten lassen sich auf geringem Bauraum besonders einfach gegeneinander drehbare und/oder verschiebbare Elemente realisieren.

In einer weiteren Ausführungsform der Erfindung beträgt die betragsmäßige Differenz des ersten Winkels und des zweiten Winkels kleiner oder gleich 360° geteilt durch die Anzahl der Sektoren, d.h. auch mit einer Drehung von weniger als 180° können die notwendigen Kräfte aufgebracht werden.

In noch einer weiteren Ausführungsform der Erfindung ist weiterhin ein Hebel zum Öffnen der Klemme vorgesehen ist, sodass ein fluidführender Kanal in die Klemme eingelegt werden kann.

Durch den Hebel kann auch im stromlosen Zustand die Klemme geöffnet werden, sodass ein fluidführender Kanal eingelegt werden kann.

Gemäß einer weiteren Ausführungsform der Erfindung wird weiterhin ein Elektromagnet bereitgestellt, der im Fall einer Aktivierung so auf zumindest einen der Permanentmagnete einwirkt, dass der Arbeitszustand gehalten wird, d.h. der Elektromagnet ist so ausgestaltet, dass der Durchfluss durch den fluidführenden Kanal ermöglicht wird.

Gemäß einer Weiterbildung der Erfindung wirkt der Elektromagnet im Fall einer Aktivierung so zumindest auf einen der Permanentmagnete ein, dass der Arbeitszustand erreicht wird, d.h., der Elektromagnet ist so ausgestaltet, dass aus dem Ruhezustand in den Arbeitszustand geschaltet werden kann.

Diese Ausführungsform eignet sich besonders bei Klemmen für Kanäle, durch die medizinische Flüssigkeiten wie Blut oder Infusionsflüssigkeiten in oder von einem Patienten geleitet werden. Dabei kann die Klemme als Teil beispielsweise einer Blutbehandlungsmaschine, eines Peritonealdialysegeräts oder einer Infusionspumpe ausgeführt sein. Sollte es in solchen Geräten zu einer Fehlersituation kommen, insbesondere bei Unterbrechung der elektrischen Versorgung des Gerätes, ist im Allgemeinen ein sicherer Zustand herbeizuführen, bei der die Fluidverbindung mit dem Patienten unterbrochen wird. Ein als Elektromagnet ausgestaltetes Halteelement, das durch eine Aktivierung mittels Stromaufnahme vom Ruhezustand in den Arbeitszustand schaltet, würde bei Stromausfall zwangsläufig vom Arbeitszustand in den Ruhezustand schalten und den Durchfluss durch den Kanal unterbrechen.

Gemäß einer Weiterbildung der Erfindung wird der erste Permanentmagnet relativ zum zweiten Permanentmagnet beim Übergang aus dem Ruhezustand in den Arbeitszustand auf einer schraubenartigen Bahn geführt.

Somit können unterschiedliche Wegprofile bereitgestellt werden.

In einer weiteren Ausführungsform stellt eine stößelartige Ausformung die Schließkraft zur Verfügung, wobei die Kraftkennlinie des Magnetpaars, gebildet aus dem ersten Permanentmagneten und dem zweiten Permanentmagneten, bei der die Anziehungskraft bei geringem Abstand der Permanentmagneten groß ist, umgesetzt wird in eine Kraftkennlinie der Schließkraft, die groß ist, wenn die Klemme geschlossen ist.

Weitere vorteilhafte Ausgestaltungen sind Gegenstand der abhängigen Ansprüche und der ausführlichen Beschreibung.

Kurzdarstellung der Figuren

Nachfolgend wird die Erfindung näher unter Bezugnahme auf die Figuren erläutert werden. In diesen zeigt:

Fig. 1 eine beispielhafte Orientierung von polymagnetisierten Permanentmagneten in einem ersten Zustand gemäß Ausführungsformen der Erfindung,

Fig. 2 eine beispielhafte Orientierung von polymagnetisierten Permanentmagneten in einem zweiten Zustand gemäß Ausführungsformen der Erfindung,

Fig. 3 eine beispielhafte Schnittdarstellung von Elementen einer erfindungsgemäßen Klemme in einem ersten Zustand,

Fig. 4 eine beispielhafte Schnittdarstellung von Elementen einer erfindungsgemäßen Klemme in einem zweiten Zustand,

Fig. 5 eine beispielhafte Explosionsdarstellung von Elementen einer erfindungsgemäßen Klemme, und

Fig. 6 ein Kraft-Weg-Diagramm von Schraubklemmen aus dem Stand der Technik sowie gemäß Ausführungsformen der Erfindung.

Detaillierte Beschreibung

Nachfolgend wird die Erfindung eingehender unter Bezugnahme auf die Figuren dargestellt werden. Dabei ist anzumerken, dass unterschiedliche Aspekte beschrieben werden, die jeweils einzeln oder in Kombination zum Einsatz kommen können, d.h. jeglicher Aspekt kann mit unterschiedlichen Ausführungsformen der Erfindung verwendet werden, soweit nicht explizit als reine Alternative dargestellt.

Weiterhin wird nachfolgend der Einfachheit halber in aller Regel immer nur auf eine Entität Bezug genommen werden. Soweit nicht explizit vermerkt, kann die Erfindung aber auch jeweils mehrere der betroffen Entitäten aufweisen. Insofern ist die Verwendung der Wörter "ein", "eine" und "eines" nur als Hinweis darauf zu verstehen, dass in einer einfachen Ausführungsform zumindest eine Entität verwendet wird.

Gemäß der Erfindung wird eine Klemme 1 bereitgestellt.

Die Klemme 1 blockiert in einem Ruhezustand den Durchfluss durch Zusammendrücken eines eingelegten fluidführenden Kanals L. In einem Arbeitszustand der Klemme 1 hingegen wird durch Rücknahme des Zusammendrückens der Durchfluss durch den eingelegten fluidführenden Kanal L ermöglicht.

D.h. die Klemme 1 kann so geartet sein, dass die Klemme 1 einen fluidführenden Kanal L aufnehmen kann, wobei die Klemme 1 in einem Ruhezustand den aufgenommenen fluidführenden Kanal L zusammendrücken kann, und dass die Klemme 1 in einem Arbeitszustand einen fluidführenden Kanal so aufnehmen kann, dass er nicht zusammengedrückt wird.

Hierzu weist die Klemme 1 einen ersten Magneten, d.h. einen Permanentmagneten, M1 und einen zweiten Magneten, d.h. einen Permanentmagneten, M2 auf, wobei die Magneten M1, M2 so zueinander beabstandet angeordnet sind, dass die Magneten M1, M2 relativ zueinander zumindest um einen Winkel drehbar sind, wobei die relative Drehung der Magneten M1, M2 zueinander zu einer Verringerung des Abstandes d führt, wobei in einem ersten Winkel der Ruhezustand und einem zweiten Winkel der Arbeitszustand zur Verfügung gestellt wird.

Insbesondere können die Permanentmagnete M1, M2 in einer Ausführungsform auch relativ zueinander beweglich so angeordnet sein, dass die Permanentmagneten M1, M2 relativ zueinander zumindest um einen Winkel drehbar sind und dass die Permanentmagneten M1, M2 linear relativ zueinander bewegbar sind, sodass der Abstand d zwischen den Permanentmagneten M1, M2 variieren kann, wobei eine Drehung der Permanentmagneten M1, M2 relativ zueinander zu einer Veränderung der relativen Anordnung der Pole der Permanentmagnete M1, M2 zueinander führt, welche wiederum eine Veränderung der magnetischen Anziehungskräfte zwischen den Permanentmagneten M1, M2 bewirkt, infolgedessen eine Veränderung des Abstandes d der Permanentmagnete M1, M2 erfolgt, wobei die Klemme 1 in einer ersten relativen Drehstellung der Permanentmagnete M1, M2 zueinander einen Ruhezustand und einer zweiten relativen Drehstellung der Permanentmagnete M1, M2 zueinander einen Arbeitszustand einnimmt.

In anderen Worten ist eine Klemme nach dieser Ausführungsform so geartet,

-   dass sie einen fluidführenden Kanal aufnehmen kann,
-   dass sie in einem Ruhezustand einen aufgenommen fluidführenden Kanal (L) zusammendrücken kann
-   und dass sie in einem Arbeitszustand einen fluidführenden Kanal so aufnehmen kann, dass er nicht zusammengedrückt wird,
-   wobei die Klemme (1) einen ersten Permanentmagneten (M1) und einen zweiten Permanentmagneten (M2) aufweist,
-   wobei die Magneten M1, M2 relativ zueinander beweglich angeordnet sind, sodass die Magneten (M1, M2) relativ zueinander zumindest um einen Winkel drehbar sind und dass die Magneten (M1, M2) linear relativ zueinander bewegbar sind, sodass der Abstand zwischen den Magneten variieren kann,
-   wobei eine Drehung der Magnete (M1, M2) relativ zueinander zu einer Veränderung der relativen Anordnung der Pole der Magnete zueinander führt, welche wiederum eine Veränderung der magnetischen Anziehungskräfte zwischen den Magneten bewirkt, infolgedessen eine Veränderung des Abstands (d) der Magnete erfolgt,
-   wobei die Klemme in einer ersten relativen Drehstellung der Magnete zueinander einen Ruhezustand und in einer zweiten relativen Drehstellung der Magnete zueinander einen Arbeitszustand einnimmt.

Ein fluidführender Kanal L kann z.B. eine Schlauchleitung oder ein in eine Kassette eingelassenen Kanal, der durch eine flexible Wandung begrenzt ist, sein.

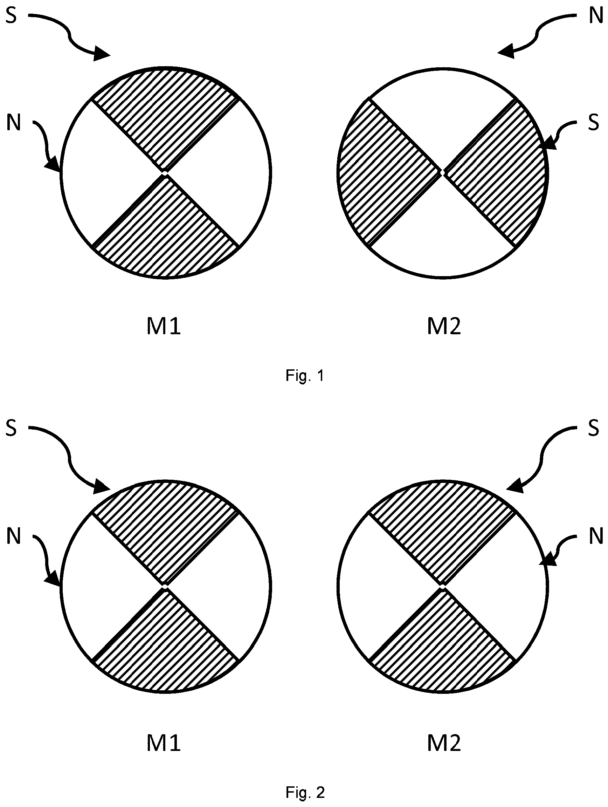

Dies soll nun an Hand von den Figuren 1-5 näher erläutert werden. In diesen ist eine beispielhafte Ausführungsform der erfindungsgemäßen Klemme 1 aufgezeigt, wobei nicht alle Elemente sichtbar sind bzw. dargestellt werden.

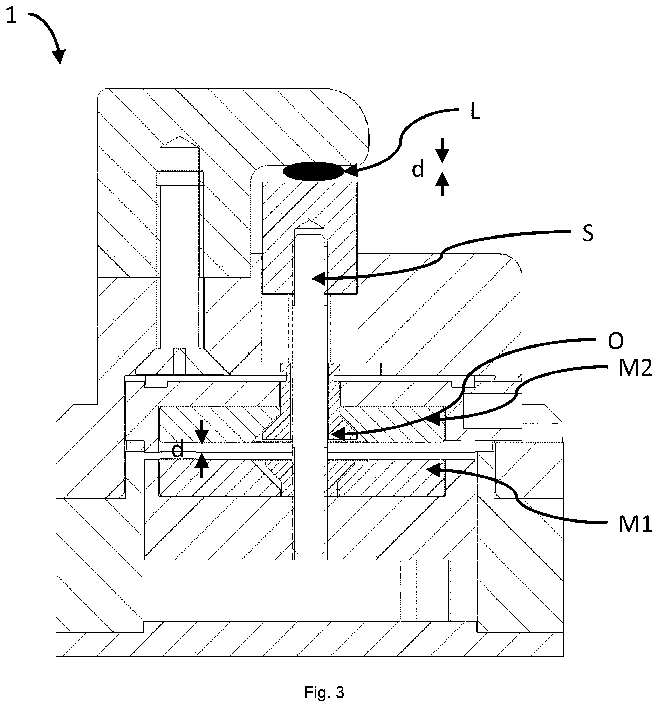

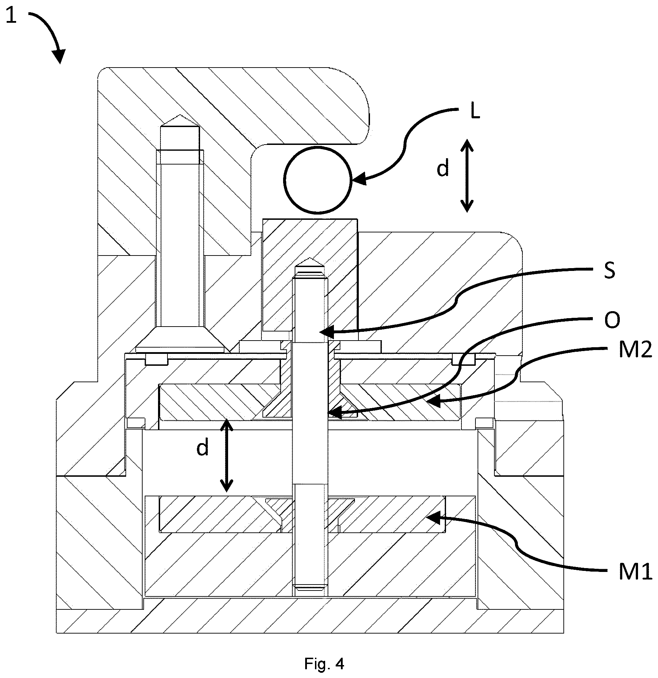

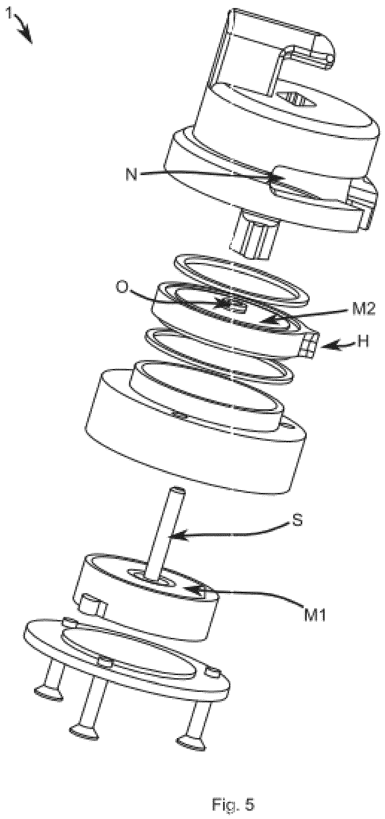

In Figur 3 und Figur 4 ist eine erfindungsgemäße Klemme 1 in einem Schnitt dargestellt. Die hierzu korrespondierende Explosionsdarstellung ist in Figur 5 gezeigt. In der Figur 3 ist eine Ausführung mit polymagnetisierter Permanentmagneten dargestellt.

Polymagnetisierte Permanentmagnete können in unterschiedlichen Ausführungen erworben werden. Polymagnetisierte Magnete weisen dabei eine (gerade) Vielzahl von Sektoren unterschiedlicher Magnetisierung (Nord / Süd) auf. D.h. neben einem Sektor mit einem magnetischen Nordpol N befindet sich ein Sektor mit einem magnetischen Südpol S. Solche polymagnetisierten Magnete sind z.B. von der Firma Correlated Magnetics Research LLC, Huntsville, AL 35806 USA erhältlich.

Wie allgemein bekannt ist, ziehen sich ungleiche Magnetpole an, während sich gleiche Magnetpole abstoßen.

In der Verwendung der Erfindung sind z.B. zwei scheibenartige Permanentmagnete M1, M2 verbaut, wobei jede Scheibe mindestens einen Südpol S und einen Nordpol N aufweist. Im Beispiel der Figur 1 und 2 weisen die Permanentmagnete M1 und M2 jeweils zweimal einen Südpol und zweimal einen Nordpol auf.

Wird nunmehr der Permanentmagnet M1 wie in Figur 1 gezeigt genau über dem in Figur 1 gezeigten Permanentmagneten M2 angeordnet, so ziehen sich die jeweiligen Pole an, und die Permanentmagnete M1, M2 sind bestrebt, ihren Abstand d zu verringern.

Wird hingegen der Permanentmagnet M1 wie in Figur 2 gezeigt genau über dem in Figur 2 gezeigten Permanentmagneten M2 angeordnet, so stoßen sich die jeweiligen Pole ab, und die Permanentmagnete M1, M2 sind bestrebt Ihren Abstand d zu vergrößern.

Werden die Permanentmagnete M1 und M2 zwischen diesen Extremen der Figur 1 und Figur 2 angeordnet, so überwiegt entweder der Abstoßungseffekt oder aber der Anziehungseffekt, bzw. an einem Punkt heben sich die Effekte bezogen auf die Anordnung auch auf.

Obwohl in den Figuren 1-5 rotationssymmetrische Permanentmagnete M1, M2 dargestellt sind, ist die Erfindung nicht hierauf beschränkt.

Durch geeignete Halterung und Führung kann nunmehr eine Zwangsbahn vorgeschrieben werden.

Dabei wird in Figur 3 ein Ruhezustand gezeigt, in dem durch die Anziehung der Permanentmagneten M1, M2 der Abstand klein wird und dabei der eingelegte fluidführende Kanal L zusammengedrückt wird, sodass der Durchfluss unterbunden wird.

In Figur 4 hingegen sind die Permanentmagneten M1, M2 gegenüber dem Ruhezustand verdreht und durch Führung auf einer Zwangsbahn ist der Abstand d vergrößert. Da mit zunehmender Verdrehung hin zum Arbeitszustand die Kraft zwischen den beiden Permanentmagneten M1, M2 abnimmt, sinkt auch die notwendige Kraft zum Halten eines so getrennten Zustandes.

In der dargestellten Ausführungsform ist z.B. der Permanentmagnet M1 in einer Nut gehalten, sodass er nur eine Translationsbewegung in vertikaler Richtung ausführen kann. Der Permanentmagnet M2 ist ebenfalls in einer Nut N gehalten, sodass er nur eine Rotationsbewegung ausführen kann, wobei die Drehachse des zweiten Permanentmagneten M2 mit der vertikalen Richtung der Translationsbewegung des ersten Permanentmagneten M1 zusammenfällt. Hierdurch wird eine beispielhafte Zwangsbahn vorgeschrieben.

In den Figuren ist eine Ausführungsform gezeigt, bei der das Federelement durch ein Paar (vorzugsweise polymagnetisierter) Permanentmagnete bereitgestellt wird. Die Permanentmagnete M1, M2 sind dabei so gelagert oder geführt, dass einer der Permanentmagnete - hier Permanentmagnet M1 - eine Bewegung in z-Richtung ausführen kann und zumindest einer der Permanentmagnete - hier Permanentmagnet M2 - eine Drehbewegung ausführen kann. Andere Bewegungskomponenten / Zwangsbahnen lässt die Lagerung/ Führung der Permanentmagnete M1, M2 nicht zu. D.h. in der dargestellten Ausführungsform kann der erste Permanentmagnet M1 eine Bewegung in z-Richtung ausführen, aber keine Drehbewegung, der zweite Permanentmagnet M2 kann eine Drehbewegung ausführen, aber keine Bewegung in z-Richtung.

In einer alternativen Ausführungsform ist der erste Permanentmagnet M1 so gelagert/ geführt, dass er eine Drehbewegung und eine Bewegung in z-Richtung ausführen kann. Der zweite Permanentmagnet M2 kann in dieser Ausführungsform so fixiert sein, dass weder eine Drehbewegung und noch eine Bewegung in z-Richtung möglich sind.

Beiden Ausführungsformen ist gemeinsam, dass die Bewegung der Permanentmagneten M1, M2 relativ zueinander sowohl eine z-Komponente als auch eine Rotationskomponente aufweist.

Es sei angemerkt, dass eine solche Zwangsbahn nicht notwendigerweise "linear" gestaltet sein muss. Vielmehr kann die Zwangsbahn jegliche geeignete Form aufweisen, um Haltekräfte bereitzustellen.

Insbesondere kann die Zwangsbahn auch so ausgestaltet sein, dass nicht nur Arbeitszustand und Ruhezustand ausgebildet sind, sondern dass auch ein oder mehrere Zwischenzustände ermöglicht werden, um eine einstellbare Ventilfunktion zu realisieren.

Ohne Beschränkung der Allgemeinheit kann das Fluid im fluidführenden Kanal L eine Flüssigkeit, wie z.B. Dialysat, (arterielles oder venöses) Blut oder aber auch ein Gas sein.

In den Figuren ist eine Ausführungsform mit einer stößelartigen Ausformung gezeigt. In diesen wird der in vertikale Richtung (in Bezug auf die Darstellung) stößelartigen Ausformung S durch den in vertikale Richtung bewegbaren Magneten M1 betätigt. Dabei ist die stößelartige Ausformung S durch eine Öffnung O durch den zweiten Magneten M2 hindurchgeführt. Die stößelartige Ausformung S kann dabei die Schließkraft z.B. auf eine Klemmschneide übertragen, d.h. die wechselseitigen (Anziehungs-) Kräfte des ersten Magneten M1 und des zweiten Magneten M2 werden in eine Schließkraft umgesetzt. Die Schließkraft wird nicht mehr durch eine (ermüdbare) Feder zur Verfügung gestellt. Die Kraftkennlinie des Magnetpaars gebildet aus dem ersten Magneten M1 und zweiten Magneten M2, bei der die Anziehungskraft bei geringem bis minimalem Abstand der Magneten groß bis maximal ist, wird umgesetzt in eine Kraftkennlinie der Schließkraft, die groß bis maximal ist, wenn die Klemme geschlossen ist. Dieser Effekt beruht auf der Übertragung der Schließkraft mittels des Stößels.

Obwohl vorstehend eine Anordnung beschrieben wurde, bei der der fluidführende Kanals L sich nicht zwischen den Magneten befindet, sondern ein stößelartige Ausformung S von dem Magneten M1 in Richtung eines Anschlages gedrückt wird und dabei den dazwischen eingelegten fluidführende Kanal L zusammendrückt, ist dies nicht die einzig mögliche Ausgestaltung. Vielmehr kann der fluidführende Kanal auch zwischen den Magneten M1, M2 angeordnet sein. Dabei sei zudem angemerkt, dass die Zuordnung von Ruhezustand als auch Arbeitszustand auch anders gewählt sein kann, sodass der Ruhezustand den fluidführenden Kanal L freigibt und der Arbeitszustand den Fluidfluss im fluidführender Kanal L unterbricht, d.h., die Erfindung ist nicht auf eine bestimmte Form festgelegt, sondern kann in jeglicher Anwendung eingesetzt werden.

In einer Ausführungsform der Erfindung ist der erste Magnet M1 und/oder der zweite Magnet M2 ein polymagnetisierter Magnet, wobei ein polymagnetisierter Magnet eine erste Anzahl $n_N$ von Sektoren einer ersten Magnetisierung N und eine zweite Anzahl $n_S$ von Sektoren einer zweiten Magnetisierung S aufweist. In aller Regel ist die Anzahl der Sektoren gleich, d.h. $n=n_S=n_N$. In einer Ausführungsform erfolgt die Aufprägung des Magnetisierungsmusters mit dem polymagnetisierten Magnet (www.polymagnet.com) Verfahren.

Der Winkelbereich ist in aller Regel sinnvoll zwischen den beiden Extremstellungen (analog zu Figur 1 und Figur 2) befindlich. Allerdings kann es auch sinnvoll sein, den Bereich kleiner zu gestalten. In aller Regel ist der Winkelbereich der Drehung, d.h. die betragsmäßige Differenz des ersten Winkels $\alpha_{Max}$ und des zweiten Winkels $\alpha_{Min}$ ist kleiner oder gleich 360° geteilt durch die Anzahl der Sektoren n, d.h. $\delta \leq \frac{|\alpha_{Max}-\alpha_{Min}|}{n}$.

In der Ausführungsform der Figur 5 ist weiterhin ein Hebel H zum mechanischen Öffnen der Klemme 1 vorgesehen, sodass ein fluidführender Kanal L in die Klemme 1 eingelegt werden kann. Der Hebel H kann z.B. an einem der Magnete M1, M2, bzw. deren Halterung, angeordnet sein. Im Beispiel der Figur 5 ist der Hebel H an der Halterung des Magneten M2 angeformt und bewegt sich in einer Nut N, d.h. dem Hebel H kommt zugleich die Funktion der Führung auf der Zwangsbahn zu.

Auch im stromlosen Zustand kann die Klemme geöffnet werden und ein fluidführender Kanal L eingelegt oder entnommen werden.

Ohne weiteres kann nun ein Elektromagnet bereitgestellt werden, der an geeigneter Stelle an oder in der Klemme 1 angeordnet ist, der im Fall einer Aktivierung so zumindest auf einen der Magneten M1, M2 einwirkt, dass der Arbeitszustand gehalten wird d.h. der aktivierte Elektromagnet wirkt der Anziehungskraft entgegen und hält den fluidführenden Kanal L offen.

Ohne weiteres kann der Elektromagnet auch so dimensioniert werden, dass er zumindest für eine Schaltzeit zur Öffnung der Klemme im Fall einer Aktivierung so zumindest auf einen der Magneten M1, M2 einwirkt, dass der Arbeitszustand erreicht wird. Nach Erreichen des Arbeitszustandes kann der Strom durch den Elektromagneten reduziert werden, da nun geringere Kräfte aufzubringen sind. Beispielsweise kann durch Microschalter das Erreichen einer bestimmten Position eines Elementes der Klemme 1 zu einer Stromreduktion verwendet werden, und/oder eine Stromreduktion wird nach Ablauf einer bestimmten Zeit vorgenommen. Offensichtlich kann auch überwacht werden, ob eine bestimmte Position nach Ablauf einer bestimmten Zeit erreicht wurde. Wird die Position nicht erreicht, kann von einer Fehlfunktion ausgegangen werden, die z.B. signalisiert werden könnte.

Wie bereits zuvor beschrieben kann die Zwangsbahn unterschiedlich gestaltet sein. Besonders einfach ist jedoch die Zwangsbahn dadurch gekennzeichnet, dass die relative Bahn vom ersten Magnet M1 in Bezug zum zweiten Magnet M2 beim Übergang aus dem Ruhezustand in den Arbeitszustand auf einer schraubenartigen Bahn abgebildet werden kann.

Die Anziehungskraft der beiden Magneten M1, M2 zueinander stellt in der beispielhaften Anordnung eine Schließkraft der Klemme 1 zur Verfügung. Das aufgeprägte Magnetisierungsmuster bewirkt, das der verbleibende Freiheitsgrad der Bewegung der Magnete M1, M2 zueinander eine schraubenförmige Komponente erhält.

Der Magnet M1, für den eine Bewegung in der z-Richtung möglich ist, ist mit dem Klemmelement verbunden, welches ein Zuklemmen des fluidführenden Kanals L ermöglicht. Der Magnet M2, für den die Drehbewegung möglich ist, ist mit dem Halteelement verbindbar.

Beide Freiheitsgrade der Bewegung führen in Kombination zu einer Schraubenartigen Relativbewegung. Aufgrund des Schraubencharakters der Beziehung zwischen der z-Komponente der Bewegung des Klemmelements und der Winkelbewegung des Magneten M2bzw. dem damit verbindbaren Halteelement, wird erreicht, dass die Haltekräfte am Halteelement in der Offen-Stellung deutlich geringer sind, als die Klemmkräfte in der Geschlossen-Stellung.

Fig. 6 zeigt das Kraft-Weg Diagramm einer herkömmlichen, mit einer mechanischen Feder gespannten Klemme (durchgezogene Linie) und einer Klemme 1 gemäß Ausführungsformen der Erfindung (gestrichelte Linie). Bei einem Weg von s=0 mm gibt die Klemme 1 den fluidführenden Kanal L frei. Bei der herkömmlichen Klemme wird dabei eine Feder durch ein Halteelement unter Spannung in dieser Stellung gehalten. Gibt das Halteelement die Feder frei, entspannt sich die Feder bis zum kompletten Verschluss des fluidführenden Kanals (hier bei s=6mm), wobei die Restspannung der Feder einen ausreichenden Verschluss in der Geschlossen-Stellung sicherstellt. Bei der Ausführung mit polymagnetisierten Magneten M1, M2 wird einerseits eine vergleichbare Verschlusskraft in der Geschlossen-Stellung erreicht, während andererseits für die Haltekraft in der Offen-Stellung eine deutliche kleinere Kraft durch das Halteelement aufgebracht werden muss. D.h. da nunmehr geringer Kräfte zum Offenhalten des fluidführenden Kanals L notwendig sind, wird auch der Energieverbrauch der Klemme 1 gegenüber herkömmlichen Klemmen reduziert.

Das Einsetzen des fluidführenden Kanals L kann durch manuelle Öffnung der Klemme 1 mit einem mit dem Halteelement verbundenen Hebel H erfolgen. In der Offenstellung wird das Halteelement z.B. mit einem Elektromagneten gehalten. Durch die geringen Haltekräfte kann der Elektromagnet kleiner dimensioniert und leichter sein sowie einen geringeren Haltestrom erfordern, was mit einem geringeren Energieverbrauch verbunden ist.

Wird der Haltestrom abgeschaltet, folgen die beiden Magneten einer Anziehungsbewegung zueinander entlang des schraubenförmigen Bewegungspfads und das Klemmelement geht in die Geschlossen-Stellung.

Diese Ausführungsform eignet sich besonders bei Klemmen 1 für fluidführende Kanäle L, durch die medizinische Flüssigkeiten wie Blut oder Infusionsflüssigkeiten in oder von einem Patienten geleitet werden. Dabei kann die Klemme 1 als Teil beispielsweise einer Blutbehandlungsmaschine, eines Peritonealdialysegeräts oder einer Infusionspumpe ausgeführt sein. Sollte es in solchen Geräten zu einer Fehlersituation kommen, insbesondere bei Unterbrechung der elektrischen Versorgung des Gerätes oder der Klemme 1, ist im Allgemeinen ein sicherer Zustand herbeizuführen, bei der die Fluidverbindung mit dem Patienten unterbrochen wird. Ein als Elektromagnet ausgestaltetes Halteelement, das durch eine Aktivierung mittels Stromaufnahme vom Ruhezustand in den Arbeitszustand schaltet, würde bei Stromausfall zwangsläufig vom Arbeitszustand in den Ruhezustand schalten und den Durchfluss durch den fluidführenden Kanal L damit unterbrechen und den sicheren Zustand erreichen

In weiteren Ausführungsformen weißt eine erfindungsgemäße Klemme 1 reibungsmindernde Elemente wie beispielsweise eine oder mehrere Gleitbuchsen O auf. Eine solche Gleitbuchse O könnte beispielsweise um eine stößelartige Ausformung S, z.B. um einen Stift oder Stempel S, angeordnet sein, welcher durch diese Buchse O geführt ist, und dessen Bewegung mit einer Drehung der beiden Permanentmagnete M1, M2 relativ zueinander und einer Veränderung des Abstands der Permanentmagnete zueinander einhergeht. Besonders vorteilhaft sind dabei Materialpaarungen zwischen Gleitbuchse O und darin gelagertem Stößel / Stift / Stempel S, welche eine möglichst geringe Haftreibung in Ruhe aufweisen, d.h. welche keinen sogenannten Stick-Slip-Effekt aufweisen, also ein möglichst geringes Losbrechmoment aufweisen. Ein Beispiel für eine solche Materialpaarung ist beispielsweise ein Stößel / Stift / Stempel S, welcher in einer Gleitbuchse O aus Kunststoff angeordnet ist. Besonders Vorteilhaft sind in dieser Paarung Kunststoffe mit Beimischungen von PTFE, Graphit oder Bornitrit. Stähle können insbesondere rostfreie Stähle sein. Weiterhin kommen Keramik und Siliziumcarbid als Materialen für Gleitbuchsen O in Frage. Eine solche Gleitbuchse O ermöglicht vorteilhaft eine reibungsärmere Bewegung eines darin gelagerten Stößels / Stifts / Stempels S, welcher direkt mit einem Verschlussglied / Klemmglied der Klemme 1 verbunden sein kann. Dadurch werden besonders vorteilhaft schnellere Verschlusszeiten, weniger Verschleiß und eine höhere Lebensdauer einer solchen Klemme 1 ermöglicht.

In einer Weiterbildung weißt eine erfindungsgemäße Klemme 1 statt einer Gleitbuchse O ein oder mehrere Gleitelemente auf, welche um ein bewegliches Teil angeordnet sind. Dabei kann es besonders vorteilhaft sein, nicht um den gesamten Umfang z.B. eines beweglichen Stößels / Stifts / Stempels S Gleitelemente anzuordnen, sondern nur in Kreisausschnitten, welche insgesamt weniger als 360 Grad bedecken. Dabei kann besonders vorteilhaft der Bedarf an Gleitelementmaterial gesenkt werden, indem stückweise Teile eines Umfangs um ein darin gleitfähiges bewegliches Teil mit Gleitelementen ausgestattet sind.

Besonders vorteilhaft sind die Permanentmagnete M1, M2 der Klemme 1 in einem Gehäuse aus einem nichtmagnetischen Material wie beispielsweise Aluminium oder Kunststoff angeordnet.

Man kann Aspekte der Erfindung wie folgt zusammenfassen: Durch das Paar von relativ zueinander beweglichen Permanentmagnete M1, M2 wird mit einer Drehbewegung eine zusätzliche Linearbewegung - z.B. normal zur Drehebene - ausgelöst. Eine relative Drehbewegung führt durch die magnetische Kopplung der Permanentmagnete M1, M2 miteinander zu einer relativen Hubbewegung, d.h. der lineare Abstand zwischen den Permanentmagneten M1, M2 verändert sich infolge einer relativen Drehbewegung.

Dieser Vorgang ist reversibel. Eine relative Drehbewegung in einem ersten Drehsinn führt zu einer Verringerung des Abstands der Permanentmagnete M1, M2 und eine relative Drehbewegung in einem zweiten (entgegengesetzten) Drehsinn führt zu einer Vergrößerung des Abstands.

Erfindungsgemäß kann die Umsetzung einer Drehbewegung in eine zusätzliche Hubbewegung erreicht werden, indem die Permanentmagnete M1, M2 so gestaltet, beweglich gelagert und angeordnet sind, dass in einer ersten Drehstellung in Summe die Anziehung zwischen den Permanentmagneten M1, M2 geringer ist als für eine zweite Drehstellung. Das kann beispielsweise dadurch erreicht werden, dass die Abstände voneinander anziehenden Polen im Mittel in der ersten Drehstellung größer sind als in der zweiten Drehstellung. Es kann aber stattdessen oder zusätzlich über die Anzahl, die Form und die Gestaltung der Pole erfolgen. Für eine solche relative Drehbewegung und für ein Halten einer bestimmten Drehstellung muss wesentlich weniger elektrische Energie aufgewendet werden, als es bei bekannten Klemmen der Fall gewesen ist, um eine Klemme in geöffneter Stellung zu halten.

## Patentansprüche

1. Klemme (1), welche so geartet ist,

   • dass die Klemme (1) einen fluidführenden Kanal (L) zwischen einem Klemmelement und einem Haltelement aufnehmen kann, wobei der fluidführende Kanal (L) als eine Schlauchleitung für medizinische Flüssigkeiten ausgestaltet ist,
   • dass die Klemme (1) in einem Ruhezustand den aufgenommenen fluidführenden Kanal (L) zusammendrücken kann
   • und dass die Klemme (1) in einem Arbeitszustand den fluidführenden Kanal (L) so aufnehmen kann, dass der fluidführende Kanal (L) nicht zusammengedrückt wird,
   • wobei die Klemme (1) einen ersten Permanentmagneten (M1) und einen zweiten Permanentmagneten (M2) aufweist,
   • wobei die Permanentmagneten (M1, M2) so in einem Abstand (d) zueinander angeordnet sind, dass die Permanentmagneten (M1, M2) relativ zueinander zumindest um einen Winkel drehbar sind, wobei die relative Drehung der Permanentmagneten (M1, M2) zueinander zu einer Verringerung des Abstandes (d) führt, wobei in einem ersten Winkel der Ruhezustand und einem zweiten Winkel der Arbeitszustand zur Verfügung gestellt wird,
   • wobei ein erster der Permanentmagneten (M1) mit dem Klemmelement verbunden ist und wobei ein zweiter der Permanentmagneten (M2) mit dem Halteelement verbunden ist.

2. Klemme (1) nach Anspruch 1, **dadurch gekennzeichnet, dass** das Fluid eine Flüssigkeit ist.

3. Klemme (1) nach Anspruch 1 oder 2, **dadurch gekennzeichnet, dass** der erste Permanentmagnet (M1) und/oder der zweite Permanentmagnet (M2) ein polymagnetisierter Magnet ist, wobei ein polymagnetisierter Magnet eine erste Anzahl von Sektoren einer ersten Magnetisierungen (N) und eine zweite Anzahl von Sektoren einer zweiten Magnetisierung (S) aufweist.

4. Klemme (1) nach Anspruch 3, wobei die betragsmäßige Differenz des ersten Winkels und des zweiten Winkels kleiner oder gleich 360° geteilt durch die Anzahl der Sektoren ist.

5. Klemme (1) nach einem der vorhergehenden Ansprüche, **dadurch gekennzeichnet, dass** weiterhin ein Hebel (H) zum Öffnen der Klemme vorgesehen ist, sodass ein fluidführender Kanal (L) in die Klemme (1) eingelegt werden kann, wobei der Hebel (H) mit dem Halteelement verbundenen ist.

6. Klemme (1) nach einem der vorhergehenden Ansprüche, **dadurch gekennzeichnet, dass** weiterhin ein Elektromagnet bereitgestellt ist, der im Fall einer Aktivierung so zumindest auf einen der Permanentmagneten (M1, M2) einwirkt, dass der Arbeitszustand gehalten wird.

7. Klemme (1) nach Anspruch 6, **dadurch gekennzeichnet, dass** der Elektromagnet im Fall einer Aktivierung so zumindest auf einen der Permanentmagneten (M1, M2) einwirkt, dass der Arbeitszustand erreicht wird.

8. Klemme (1) nach einem der vorhergehenden Ansprüche, **dadurch gekennzeichnet, dass** der erste Permanentmagnet (M1) relativ zum zweiten Permanentmagnet (M2) beim Übergang aus dem Ruhezustand in den Arbeitszustand auf einer schraubenartigen Bahn geführt wird, wobei der zweite Permanentmagnet (M2) eine Drehbewegung und der erste Permanentmagnet (M1) eine Bewegung in senkrecht zur Drehbewegung ermöglicht, wobei beide Freiheitsgrade der Bewegung in Kombination eine schraubenartige Relativbewegung ermöglichen.

9. Klemme (1) nach einem der vorhergehenden Ansprüche, **dadurch gekennzeichnet, dass** eine stößelartige Ausformung (S) die Schließkraft der Klemme (1) zur Verfügung stellt, wobei die Kraftkennlinie des Magnetpaars, gebildet aus dem ersten Permanentmagneten (M1) und dem zweiten Permanentmagneten (M2), bei der die Anziehungskraft bei geringem Abstand der Magneten groß ist, umgesetzt wird in eine Kraftkennlinie der Schließkraft, die groß ist, wenn die Klemme geschlossen ist, wobei die stößelartige Ausformung durch den ersten Permanentmagneten (M1) betätigbar ist, wobei die stößelartige Ausformung S durch eine Öffnung (O) durch den zweiten Permanentmagneten (M2) hindurchgeführt ist.

10. Klemme (1) nach einem der vorhergehenden Ansprüche, **dadurch gekennzeichnet, dass** der fluidführende Kanal (L) zwischen den Permanentmagneten (M1, M2) angeordnet ist.


## Claims

1. A clamp (1), which is arranged such

   • that the clamp (1) can accommodate a fluid-carrying channel (L) in between a clamp element and a holding element, whereby the fluid-carrying channel (L) is arranged as a flexible line for medical fluids,
   • that the clamp (1) can compress the accommodated fluid-carrying channel in a resting state
• and that in a working state the clamp (1) can accommodate the fluid-carrying channel (L) so that the fluid-carrying channel (L) is not compressed,
• whereby the clamp (1) has a first permanent magnet (M1) and a second permanent magnet (M2),
• whereby the permanent magnets (M1, M2) being arranged at a distance (d) from one another, such that the permanent magnets can be rotated at least about an angle relative to one another, the relative rotation of the permanent magnets (M1, M2) to one another resulting in a reduction in the distance (d), the resting state being made available at a first angle, and the working state being made available at a second angle
• whereby a first of the permanent magnets (M1) is connected with the clamp element und whereby a second of the permanent magnets (M1) is connected with the holding element.

2. The clamp (1) according to claim 1, **characterized in that** the fluid is a liquid.

3. The clamp (1) according to claim 1 or 2, **characterized in that** the first permanent magnet (M1) and/or the second permanent magnet (M2) is a polymagnetized magnet, wherein a polymagnetized magnet has a first number of sectors of a first magnetization (N) and a second number of sectors of a second magnetization (S).

4. The clamp (1) according to claim 3, wherein the difference in amount between the first angle and the second angle is less than or equal to 360° divided by the number of sectors.

5. The clamp (1) according to one of the preceding claims, **characterized in that** a lever (H) is additionally provided for opening the clamp so that a fluid-carrying channel (L) can be inserted into the clamp, whereby the lever (H) is connected to the holding element.

6. The clamp (1) according to one of the preceding claims, **characterized in that** an electromagnet is additionally provided, acting on at least one of the permanent magnets (M1, M2) in the event of activation, so that the working state is maintained.

7. The clamp (1) according to claim 6, **characterized in that** the electromagnet acts on at least one of the permanent magnets in the event of activation, so that the working state is achieved.

8. The clamp (1) according to one of the preceding claims, **characterized in that** the first permanent magnet (M1) is guided on a helical path relative to the second permanent magnet (M2) in the transition from the resting state to the working state, whereby the second permanent magnet (M2) allows for a rotary motion and the first permanent magnet (M1) allows for a motion perpendicular to the rotary motion, whereby both degrees of freedom of the motions allow in combination a relative helical movement.

9. The clamp (1) according to one of the preceding claims, **characterized in that** a rod-type design (S) makes available the closing force of the clamp (1), wherein the force characteristic of the pair of magnets formed by the first permanent magnet (M1) and the second permanent magnet (M2), in which the attractive force is great when the distance between the magnets is small, is converted into a force characteristic of the closing force, which great when the clamp is closed, whereby the rod-type design is guided through an opening (O) within the second permanent magnet (M2).

10. The clamp (1) according to one of the preceding claims 1, **characterized in that** the fluid-carrying channel (L) is arranged between the permanent magnets (M1, M2).

## Revendications

1. Camp (1), qui est agencé de sorte que

• la clamp (1) puisse accueillir un canal de transport de fluide (L) entre un élément de clamp et un élément de maintien, moyennant quoi le canal de transport de fluide (L) est agencé comme une ligne flexible pour les fluides médicaux,
• le clamp (1) peut comprimer le canal de transport de fluide (L) accueilli dans un état de repos,
• et qu'en fonctionnement, le clamp (1) peut accueillir le canal de transport de fluide (L) de sorte que le canal de transport de fluide (L) ne soit pas comprimé,
• moyennant quoi le clamp (1) comporte un premier aimant permanent (M1) et un deuxième aimant permanent (M2),
• moyennant quoi les aimants permanents (M1, M2) sont disposés à une distance (d) l'un de l'autre, de sorte que les aimants permanents (M1, M2) puissant être tournés d'environ au moins un angle l'un par rapport à l'autre, le mouvement relatif des aimants permanents (M1, M2) l'un par rapport à l'autre entraînant une réduction de la distance (d), l'état de repos étant rendu disponible à un premier angle et l'état de fonctionnement étant rendu disponible à un deuxième angle,
• moyennant quoi un premier des aimants permanents (M1) est connecté à l'élément de clamp et moyennant quoi un deuxième des aimants permanents (M2) est connecté à l'élément de maintien.

2. Pince (1) selon la revendication 1, **caractérisé en ce que** le fluide est un liquide.

3. Pince (1) selon la revendication 1 ou 2, **caractérisé en ce que** le premier aimant permanent (M1) et/ou le deuxième aimant permanent (M2) est un aimant polymagnétisé, dans lequel un aimant polymagnétisé a un premier nombre de secteurs d'une première aimantation (N) et un deuxième nombre de secteurs d'une deuxième aimantation (S).

4. Clamp (1) selon la revendication 3, dans lequel la différence entre le premier angle et le deuxième angle est inférieure ou égale à 360° divisé par le nombre de secteurs.

5. Clamp (1) selon une des revendications précédentes, **caractérisé en ce qu'**un levier (H) est prévu additionnellement pour ouvrir le clamp (1) de sorte qu'un canal de transport de fluide (L) puisse être inséré dans le clamp, moyennant quoi le levier (H) est connecté à l'élément de retenue.

6. Clamp (1) selon une des revendications précédentes, **caractérisé en ce que** un électroaimant est prévu additionnellement, agissant sur au moins un des aimants permanents (M1, M2) en cas d'activation, de sorte que l'état de fonctionnement soit maintenu.

7. Clamp (1) selon la revendication 6, **caractérisé en ce que** l'électroaimant agit sur au moins un des aimants permanents (M1, M2) en cas d'activation, de sorte que l'état de fonctionnement soit atteint.

8. Clamp (1) selon une des revendications précédentes, **caractérisé en ce que** le premier aimant permanent (M1) est guidé sur un trajet hélicoïdal par rapport au deuxième aimant permanent (M2) dans la transition de l'état de repos à l'état de fonctionnement, moyennant quoi le deuxième aimant permanent (M2) permet un mouvement rotatif et le premier aimant permanent (M1) permet un mouvement perpendiculaire au mouvement rotatif, moyennant quoi les deux degrés de liberté des mouvement permettent en combinaison un mouvement hélicoïdal relatif.

9. Clamp (1) selon une des revendications précédentes, **caractérisé en ce que** une conception de type tige (S) rend disponible la force de fermeture du clamp (1), dans lequel la caractéristique de force de la paire d'aimants formés par le premier aimant permanent (M1) et le deuxième aimant permanent (M2), dans laquelle la force d'attraction est importante lorsque la distance entre les aimants se réduit, est convertie en une caractéristique de force de la force de fermeture, qui est importante lorsque le clamp est fermé, moyennant quoi la conception de type tige est guidée à travers une ouverture (O) à l'intérieur du deuxième aimant permanent (M2).

10. Clamp (1) selon une des revendications précédentes, **caractérisé en ce que** le canal de transport de fluide (L) est agencé entre les aimants permanents (M1, M2).

Fig. 1

Fig. 2

Fig. 3

1

L

d

S

O

M2

d

M1

Fig. 4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2014318639 A1
- EP 1132108 A1